# EUROPÄISCHE PATENTSCHRIFT

(11) **EP 1 301 188 B1**
(45) Veröffentlichungstag und Bekanntmachung des Hinweises auf die Patenterteilung: **13.10.2004**
(21) Anmeldenummer: 01957894.7
(22) Anmeldetag: 26.06.2001
(51) Int. Cl.: A61K 31/5375, A01N 43/84, A01N 47/38

(54) **ANTHELMINTHIKA ZUR VERHINDERUNG VON PARASITÄREN INFEKTIONEN BEI MENSCH UND TIER III**
ANTHELMINTIC AGENTS FOR PREVENTING PARASITIC INFECTIONS IN HUMANS AND ANIMALS III
ANTHELMINTHIQUES POUR INHIBER DES INFECTIONS PARASITAIRES CHEZ L'HOMME ET L'ANIMAL III

(30) Priorität: 06.07.2000 DE 10032877
(43) Veröffentlichungstag der Anmeldung: 16.04.2003
(73) Patentinhaber: Bayer HealthCare AG, 51368 Leverkusen (DE)
(72) Erfinder: HARDER, Achim, 51109 Köln (DE); VON SAMSON-HIMMELSTJERNA, Georg, 30900 Mellendorf (DE); KRÜGER, Bernd-Wieland, 51567 Bergisch Gladbach (DE); MEHLHORN, Heinz, 41468 Neuss (DE); SCHMIDT, Jürgen, 40221 Düsseldorf (DE)
(86) Internationale Anmeldenummer: PCT/EP2001/007294
(87) Internationale Veröffentlichungsnummer: WO 2002/002088

(56) Entgegenhaltungen:
- DE-A- 4 018 070
- FR-A- 2 050 522

## Beschreibung

Die vorliegende Erfindung betrifft die Verwendung von als Repellentien geeigneten Wirkstoffen zur Verhinderung einer Infektion des Menschen bzw. von Tieren mit den Infektionsstadien von parasitischen Plattwürmern (Plathelminthen). Die Wirkstoffe kommen dabei auf der Haut gegen solche Stadien der Plattwürmer, sogenannte Cercarien, zum Einsatz, die durch die Haut in den Wirtskörper eindringen können.

Mehrere Arten von Plathelminthen verursachen schwerwiegende Erkrankungen von Menschen und Tieren. In tropischen Ländern führen insbesondere Infektionen mit Schistosoma-Arten zu chronischem Leiden und oft zum Tod. Wichtige Erreger sind Schistosoma mansoni, Schistosoma haematobium und Schistosoma japonicum. Betroffen sind die einheimische Bevölkerung, Touristen, Mitarbeiter von humanitären Hilfsorganisationen sowie militärisches Personal. Bei der Infektion des Menschen können die infektionsfähigen Cercarien, die sich im Wasser offener Gewässer befinden, durch die Haut in den Körper eindringen.

Ebenfalls problematisch ist in Ländern mit gemäßigtem Klima der Befall von Menschen mit Cercarien verschiedener Arten der Gattungen Trichobilharzia und Ornithobilharzia, die sich in die Haut einbohren und eine Dermatitis hervorrufen können. Solche Infekte erfolgen bei Freizeitaktivitäten an Binnengewässern oder Meeresküsten sowie bei Tätigkeiten in der Fischerei, Teichwirtschaft oder Feldbewässerung. Generell ist in vielen Situationen des täglichen Lebens der Kontakt der Haut mit u. U. kontaminiertem/infiziertem Wasser unvermeidbar.

Eine erfindungsgemäße Vorbehandlung der Haut mit anthelminthisch wirkenden Substanzen kann jedoch vor einem Eindringen der Erreger schützen.

In der Vergangenheit wurden bereits einige Verbindungen auf ihre Tauglichkeit zur Verhinderung von Infektionen mit solchen Parasiten getestet. Die bisher für die erfindungsgemäßen Zwecke beschriebenen Substanzen sind jedoch toxisch, wenn sie durch die Haut oder nach oraler Aufnahme in den Körper gelangen:

So zeigt z.B. Hexachlorophen eine abtötende Wirkung auf Cercarien von Schistosoma mansoni (Fripp, P. J. and Armstrong, F. I., The efficacy of hexachlorophene skin cleanser as a cercariae repellent. South African Med. J. 47: 1973, 526-527). Hexachlorophen kann wegen gesundheitlicher Risiken, insbesondere Leberschäden, beim Menschen nicht auf der Haut angewandt werden. Es ist giftig bei Berührung mit der Haut und beim Verschlucken, kann möglicherweise Missbildungen hervorrufen und ist eventuell krebserregend [Kommission der Europäischen Gemeinschaften, Richtlinie 93/72/EWG vom 1 .Sept. 1993, Anhang Bd. I und II (EU Gefahrstoff-Verordnung) mit Ergänzungen bis 1999, Amtsblatt der EUL258A, 36. Jahrgang, 16. Okt. 1993, Ergänzungen bis 1997].

Niclosamid wirkt gegen Eindringen von Cercarien [Bruce, J. I. et al. (1992) Efficacy of niclosamide as a potential topical antipenetrant (TAP) against cercariae of Schistosoma mansoni in monkeys. Mem. Inst. Oswaldo Cruz 87:28 1-289.] ist aber toxikologisch bedenklich, weil es möglicherweise vererbbare genetische Schäden verursachen kann (Registry of Toxic Effects of Chemical Substances, National Institute of Occupational Safety and Health). Die Anwendung auf der Haut bei Exposition in Gewässern verbietet sich durch seine umweltgefährdende Eigenschaft, da Niclosamid stark wassergefährdend ist [Umweltbundesamt (Hrsg.)Katalog wassergefährdender Stoffe. LTwS-Nr. 12. Mai 1996 mit laufenden Ergänzungen, Berlin 1996]. Daher hat bisher keine kommerzielle Anwendung gegen Cercarien beim Menschen stattgefunden.

N, N-Diethyl-m-toluamid (DEET) wirkt auf Cercarien von Schistosoma mansoni [Salafsky, B. et al. Evaluation of N, N-diethyl-m-toluamide (DEET)as a topical agent for preventing skin penetration by cercariae of Schistosoma mansoni. Am. J. Trop. Med. Hyg. 58: 1998, 828- 834). DEET besitzt jedoch einige ungünstige Eigenschaften.

Die Wirkung der bisher beschriebenen Anthelminthika gegen infektiöse Stadien von Plathelminthen wurde bisher nur an Cercarien der Art Schistosoma mansoni getestet, so dass eine Wirksamkeit dieser Mittel gegen andere Wurmarten bislang nicht nachgewiesen war.

Es wurde nun überraschenderweise gefunden, dass die erfindungsgemäße Verwendung von Wirkstoffen geeignet ist, um Mensch und Tier einen effektiven Schutz vor Infektionen mit Plathelminthen, insbesondere Schistosoma haematobium, Schistosoma japonicum, Trichobilharzia spp. und Omithobilharzia spp., aber auch Echinostoma spp. u.a. zu bieten.

Die Erfindung betrifft demnach
1. Verwendung von Verbindungen der Formel (Ia) 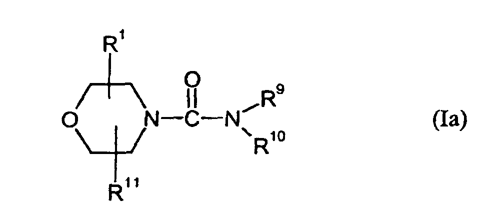
in welcher
- R¹ und R¹¹: gleich oder verschieden sind und für Wasserstoff, C₁-C₆-Alkyl, C₂-C₆-Alkenyl, C₃-C₆-Cycloalkyl oder Phenyl stehen,
- R⁹: für gegebenenfalls durch C₁-C₄-Alkoxy substituiertes C₁-C₆-Alkyl, C₂-C₆-Alkenyl, C₃-C₆-Alkinyl, C₃-C₆-Cycloalkyl, Benzyl oder Cyclohexylmethyl steht,
- R¹⁰: für Wasserstoff, gegebenenfalls durch C₁-C₄-Alkoxy substituiertes C₁-C₆-Alkyl, C₃-C₆-Cycloalkyl oder Benzyl steht,
oder R⁹ und R¹⁰ zusammen für einen gegebenenfalls durch Sauerstoff unterbrochenen und gegebenenfalls durch C₁-C₄-Alkyl substituierten Tetramethylen-, Pentamethylen-, Hexamethylen- oder Heptamethylenrest stehen, zur Abwehr von Plattwurin-Cercarien.

Besonders geeignet zum Einsatz in den erfindungsgemäßen Mittel ist die Verbindung der Formel

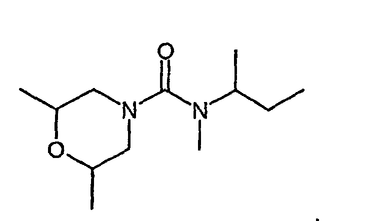

Die Verbindungen der Formel (I) und ihre Herstellung sind aus DE OS 40 18 070 bekannt.

Die erfindungsgemäß verwendeten Wirkstoffe wurden bereits speziell zur Verwendung als Repellent auf der Haut gegen Insekten und Zecken eingesetzt.

Ein wesentlicher Vorteil der Verwendung der erfindungsgemäßen Verbindungen ist deren hohe Haut-, Pflanzen- und Umweltverträglichkeit und die generell geringe Toxizität dieser Verbindungen .

Es ist weiterhin wünschenswert, beim Aufenthalt im Freien gegen Moskitos geschützt zu sein, die zum Einen als belästigend empfunden werden, zum Anderen können die Moskitos mit ihren Stichen speziell in den Tropen Krankheiten wie Malaria, verschiedene Viren, Filarien und Parasiten übertragen. Die erfindungsgemäßen Mittel ermöglichen nun die gleichzeitige Prävention vor Infektionen mit Plathelminthen und Schutz vor Moskitos mit einem Mittel. Damit wird die Notwendigkeit der gleichzeitigen Anwendung von zwei verschiedenen, möglicherweise nicht miteinander verträglichen Mitteln auf der Haut vermieden.

Die erfindungsgemäß verwendeten Mittel können neben den Wirkstoffen auch alle üblichen Hilfs- und Zusatzstoffe enthalten, welche in Formulierungen zur topikalen Applikation verwendet werden.

Die Anwendung der Wirkstoffe erfolgt direkt oder in Form von geeigneten Zubereitungen dermal oder mit Hilfe wirkstoffhaltiger Formkörper wie z.B. Streifen, Platten, Bänder, Halsbänder, Ohrmarken, Gliedmaßenbänder, Markierungsvorrichtungen.

Die dermale Anwendung geschieht z.B. in Form des Badens, Tauchens (Dippen), Sprühens (Sprayen), Aufgießens (pour-on oder spot-on), Waschens, Schamponierens, Begießens, Einpuderns.

Geeignete Zubereitungen sind:
Lösungen oder Konzentrate zur Verabreichung nach Verdünnung, Lösungen zum Gebrauch auf der Haut, Aufgussformulierungen, Gele;
Emulsionen und Suspensionen zur dermalen Anwendung sowie halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, wirkstoffhaltige Formkörper.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht.

Die Lösungen werden hergestellt, indem der Wirkstoff in einem geeigneten Lösungsmittel gelöst wird und evtl. Zusätze wie Lösungsvermittler, Säuren, Basen, Puffersalze, Antioxidantien, Konservierungsmittel zugefügt werden.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Es kann vorteilhaft sein, bei der Herstellung der Lösungen Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele, die auf die Haut aufgetragen oder aufgestrichen werden, werden hergestellt indem Lösungen, die wie oben beschrieben hergestellt worden sind, mit soviel Verdickungsmittel versetzt werden, dass eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmonobutylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Hilfsstoffe sind auch spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäurebiglycerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitanmonooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthaphosphorsäureestermonoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen werden hergestellt, indem man den Wirkstoff in einer Trägerflüssigkeit gegebenenfalls unter Zusatz weiterer Hilfsstoffe wie Netzmittel, Farbstoffe, resoxptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen zur dermalen Verabreichung unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite.

Weiterhin ist es wünschenswert, dass ein solches Schutzmittel auch noch nach längerem Wasserkontakt, beispielsweise beim Schwimmen, Kleiderwaschen oder Fischen, noch eine ausreichende Schutzwirkung zeigt. Zu diesem Zweck können die erfindungsgemäßen Mittel auch wasserabweisende bzw. wasserfeste Substanzen enthalten.

Geeignete wasserfeste Substanzen wurden bisher schon in Sonnenschutzmitteln eingesetzt, die den Benutzer gegen die UV-Strahlung der Sonne schützen sollen (z.B. US 55 18712 und US 4810489). Das Ziel war dabei, den Sonnenschutz auch aufrecht zu erhalten wenn der Benutzer schwimmen war oder heftig schwitzt etc. Sonnenschutzmittel enthaltend solche wasserfesten bzw. wasserabweisenden Substanzen und Insektenrepellentien sind bereits bekannt (US5716602). Bisher wurden jedoch noch keine Mittel, enthaltend Anthelminthika beschrieben.

Es können dementsprechend auch wasserfeste Substanzen im erfindungsgemäßen Mittel enthalten sein. Dies können fettlösliche, wasserunlösliche Stoffe sein sowie Verbindungen welche die Haftung des Mittels auf der Haut erhöhen.

In Hautschutzprodukten könnten als wasserfeste Bestandteile beispielsweise 1 bis 50 Gew.-% eines Polymers wie Polyinylpyrrolidone, Polyacrylate, Silicone etc. enthalten sein.

Die Mittel zur topischen Anwendung können als Spray, Lösung, Creme, Salbe oder schicht- bzw. filmbildende Mittel, nach den zur Herstellung von Kosmetika bekannten Verfahren (Schrader, K. (1979) Grundlagen und Rezepturen der Kosmetika. Dr. Alfred Hüthig Verlag, Heidelberg), formuliert werden.

Zur Anwendung werden die erfindungsgemäßen Formulierungen in verbrauchergerechter Menge gleichmäßig und lückenlos abdeckend auf die Haut aufgetragen.

Die erfindungsgemäß verwendeten Mittel sind selbstverständlich auch zur Anwendung am Tier geeignet, um die Infektion der Tiere mit Parasiten dieser Gattungen zu verhindern.

Die Mittel können bei Hobbytieren, wie beispielsweise Hunden und Katzen, und bei Nutztieren, beispielsweise Rinder, Schafe etc., angewendet werden.

Bei der Anwendung der erfindungsgemäßen Mittel werden im allgemeinen 0,03 bis 1 mg, bevorzugt 0,03 bis 0,1 mg und besonders bevorzugt 0,04 bis 0,06 mg des Wirkstoffes pro Quadratzentimeter Haut aufgebracht. Dadurch wird ein prophylaktischer Schutz gegen hautdurchdringende Würmer bzw. deren Vorstadien erreicht. Während eines längeren Aufenthaltes im Wasser ist der Wirkstoff wiederholt aufzutragen.

Die erfindungsgemäß Verwendung der Mittel werden durch die folgendes Beispiel illustriert.

### Biologisches Beispiel

### Wirksamkeit gegen Schistosoma mansoni-Cercarien

### [500 µl/l Endkonzentration der Wirkstoffe]

Zur Infektion wurden Schnecken (*Biomphalaria glabrata*) mit jeweils 8 Miracidien in in 10 ml Wasser über Nacht inkubiert. Cercarien wurden etwa 6 bis 9 Wochen nach Infektion gewonnen, indem die im Dunkeln gehaltenen Schnecken mit Licht bestrahlt und die danach ausschwärmenden Cercarien innerhalb von 2 Stunden gesammelt wurden.

Den Versuchsansätzen wurde soviel Cercarien-haltiges Wasser zugesetzt (1 bzw 2 ml, siehe unten), daß jeder Ansatz etwa 100 bis 150 Cercarien enthielt.

5 µl Wirkstoff wurden mit 25 µl PEG300 gründlich vermischt. Anschließend wurde 9 ml Aquarienwasser zugesetzt und der Ansatz heftig geschüttelt. Nach (zeitversetzter) Zugabe von 1 ml Cercarien-Suspension wurde jeweils ab sofort mit der Stereolupe das Überleben der Cercarien beobachtet. Zur Einteilung der Wirksamkeit der Wirkstoffe wurde folgende Einteilung verwendet : 0 = keine Wirkung über die gesamte Versuchsdauer von 120 Minuten; 1 = schwache Wirkung (Cercarien weisen eine stark verringerte Beweglichkeit auf); 2 = gute Wirkung (Cercarien sind nur noch leicht beweglich und gekrümmt); 3 = volle Wirksamkeit (Cercarien sind vollkomen regungslos)

In diesem Test wurde die Wirkung der Verbindung der folgenden Formel 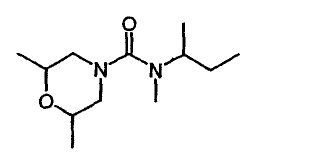 mit 1 bewertet.

## Patentansprüche

1. Verwendung von Verbindungen der Formel (Ia) 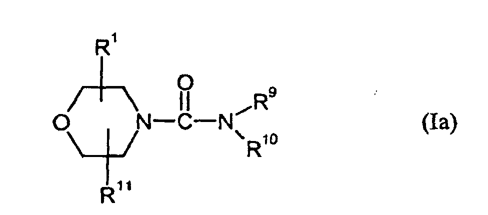 in welcher
R¹ und R¹ gleich oder verschieden sind und für Wasserstoff, C₁-C₆-Alkyl, C₂-C₆-Alkenyl, C₃-C₆-Cycloalkyl oder Phenyl stehen,
R⁹ für gegebenenfalls durch C₁-C₄-Alkoxy substituiertes C₁-C₆-Alkyl, C₂-C₆-Alkenyl, C₃-C₆-Alkinyl, C₃-C₆-Cycloalkyl, Benzyl oder Cyclohexylmethyl steht,
R¹⁰ für Wasserstoff, gegebenenfalls durch C₁-C₄-Alkoxy substituiertes C₁-C₆-Alkyl, C₃-C₆-Cycloalkyl oder Benzyl steht,
oder R⁹ und R¹⁰ zusammen für einen gegebenenfalls durch Sauerstoff unterbrochenen und gegebenenfalls durch C₁-C₄-Alkyl substituierten Tetramethylen-, Pentamethylen-, Hexamethylen- oder Heptamethylenrest stehen, zur Abwehr von Plattwurm-Cercarien.

2. Verwendung gemäß Anspruch 1 von Verbindungen der Formel (Ib) 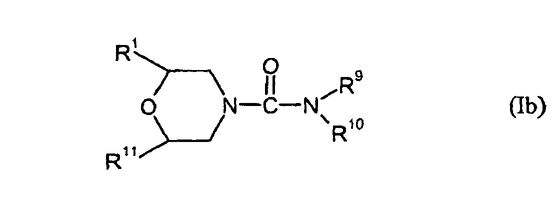 in welcher die Reste R¹, R¹¹ und R¹⁰ die in Anspruch 1 angegebene Bedeutung besitzen.

3. Verfahren zur Abwehr von Plattwurm-Cercarien, **dadurch gekennzeichnet, dass** man Verbindungen der Formel (I), definiert in Anspruch 1, auf die Plattwurm-Cercarien und/oder ihren Lebensraum einwirken lässt.

## Claims

1. Use of compounds of the formula (Ia) 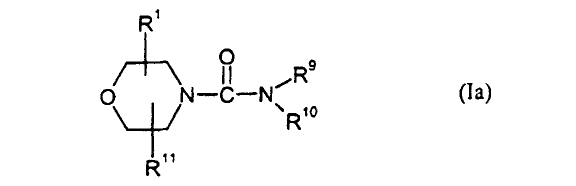 in which
R¹ and R¹¹ are identical or different and represent hydrogen, C₁-C₆-alkyl, C₂-C₆-alkenyl, C₃-C₆-cycloalkyl or phenyl,
R⁹ represents optionally C₁-C₄-alkoxy-substituted C₁-C₆-alkyl, C₂-C₆-alkenyl, C₃-C₆-alkinyl, C₃-C₆-cycloalkyl, benzyl or cyclohexylmethyl,
R¹⁰ represents hydrogen, optionally C₁-C₄-alkoxy-substituted C₁-C₆-alkyl, C₃-C₆-cycloalkyl or benzyl,
or R⁹ and R¹⁰ together represent a tetramethylene, pentamethylene, hexamethylene or heptamethylene radical which is optionally interrupted by oxygen and optionally substituted by C₁-C₄-alkyl, for deterring flatworm cercariae.

2. Use, according to Claim 1, of compounds of the formula (Ib) 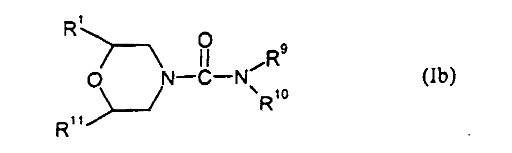 in which the radicals R¹, R¹¹ and R¹⁰ are as defined in Claim 1.

3. Method for deterring flatworm cercariae, **characterized in that** compounds of the formula (Ia) according to Claim 1 are allowed to act on the flatworm cercariae and/or their habitat.

## Revendications

1. Utilisation de composés de formule (Ia) 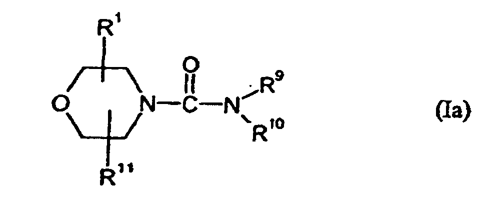 dans laquelle
R¹ et R¹¹ sont identiques ou différents et représentent l'hydrogène, un reste alkyle en C₁ à C₆, alcényle en C₂ à C₆, cycloalkyle en C₃ à C₆ ou phényle,
R⁹ représente un reste alkyle en C₁ à C₆ éventuellement substitué par un radical alkoxy en C₁ à C₄, un reste alcényle en C₂ à C₆, alcynyle en C₃ à C₆, cycloalkyle en C₃ à C₆, benzyle ou cyclohexylméthyle,
R¹⁰ représente l'hydrogène, un reste alkyle en C₁ à C₆ éventuellement substitué par un radical alkoxy en C₁ à C₄, un reste cycloalkyle en C₃ à C₆ ou benzyle,
ou bien R⁹ et R¹⁰ forment ensemble un reste tétraméthylène, pentaméthylène, hexaméthylène ou heptaméthylène éventuellement interrompu par de l'oxygène et substitué le cas échéant par un radical alkyle en C₁ à C₄,
en vue de la défense contre des cercaires de plathelminthes.

2. Utilisation suivant la revendication 1 de composés de formule (Ib) 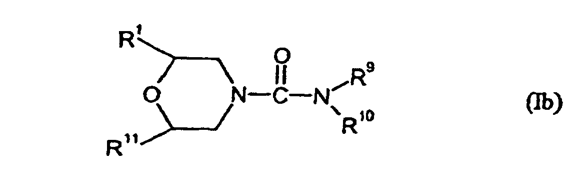 dans laquelle les restes R¹, R¹¹ et R¹⁰ ont la définition indiquée dans la revendication 1.

3. Procédé de défense contre des cercaires de plathelminthes, **caractérisé en ce qu'**on fait agir des composés de formule (I) définis dans la revendication 1, sur les cercaires de plathelminthes et/ou sur leur milieu.
